# EUROPEAN PATENT APPLICATION

(11) **EP 4 122 493 A1**
(43) Date of publication of application: **25.01.2023**
(21) Application number: 21770913.8
(22) Date of filing: 19.03.2021
(51) Int. Cl.: A61K 39/395, A61P 3/06, C07K 16/40, A61K 39/00

(54) **METHOD FOR TREATING CHOLESTEROL-RELATED DISEASES**

(30) Priority: 19.03.2020 CN 202010194665
(71) Applicant: Suzhou Suncadia Biopharmaceuticals Co., Ltd., Suzhou, Jiangsu 215126 (CN); Jiangsu Hengrui Pharmaceuticals Co., Ltd., Lianyungang, Jiangsu 222047 (CN)
(72) Inventor: ZHANG, Lianshan, Lianyungang, Jiangsu 222047 (CN); ZHU, Bo, Lianyungang, Jiangsu 222047 (CN); XIA, Jing, Lianyungang, Jiangsu 222047 (CN)
(74) Representative: dompatent von Kreisler Selting Werner - Partnerschaft von Patent- und Rechtsanwälten mbB
(86) International application number: PCT/CN2021/081705
(87) International publication number: WO 2021/185344

(57) **Abstract**

Provided in the present invention is a method for treating cholesterol-related diseases. In particular, in the present invention, an antibody combating proprotein convertase subtilisin/kexin Type 9 (PCSK9) or an antigen-binding fragment thereof is used to treat or prevent cholesterol-related diseases.

## Description

The present application claims priority to Chinese Patent Application No. CN202010194665.6 filed on Mar. 19, 2020, which are incorporated herein by reference in their entirety.

### TECHNICAL FIELD

The present disclosure relates to a method for treating or preventing cholesterol-related diseases (such as hyperlipidemia, hyperlipidemia or dyslipidemia) using an antibody or an antigen-binding fragment against proprotein convertase subtilin/kexin type 9 (PCSK9).

### BACKGROUND

Cardiovascular disease is a major disease threatening human health today. The 2016 European Society of Cardiology, together with the European Atherosclerosis Society (ESC/EAS), issued the 2016 ESC/EAS Guidelines for the Management of Dyslipidemia, which states that over 4 million people die annually in Europe due to cardiovascular diseases (CVDs). In 2009, the medical costs associated with CVDs amounted to 106 billion Euros, accounting for approximately 9% of the total medical budget of the European Union. In the United States, the direct annual cost of CVDs is expected to increase by a factor of 3 during 2010 to 2030. In China, nearly 300 million patients suffer from cardiovascular diseases and about 3.5 million patients die of various types of cardiovascular diseases annually, with the direct medical expenditure reaching 130 billion CNY, which shows an upward trend year by year. Research indicates that dyslipidemia characterized by elevated low-density lipoprotein cholesterol (LDL-C) or total cholesterol (TC) is an important risk factor for atherosclerotic cardiovascular diseases (ASCVDs). Lowering LDL-C or TC levels can significantly reduce the risk of morbidity and mortality from ASCVDs. Research shows that the annual incidence rate of heart attack, revascularization and ischemic stroke is reduced by more than 20% for decreasing by each 1.0 mmol/L of LDL-C.

At present, the treatment rate and the treatment target of dyslipidemia in China are still at a relatively low level. The clinical mainstream drugs for lowering cholesterol are statins (cholesterol synthesis inhibitors) and ezetimibe (cholesterol absorption inhibitors). The statins are HMG-CoA reductase inhibitors, and mainly achieve the purpose of reducing lipids by inhibiting cholesterol synthesis rate-limiting enzyme HMG-CoA reductase and thus reducing cholesterol synthesis. Clinical trials have demonstrated that statins can reduce mortality in the high-risk group having CVDs. Statins are effective in the treatment of early stages of disease (secondary prevention) and elevated risk without CVDs (primary prevention). The application effect of the statins in the low-risk group having CVDs is to be further researched. However, statins have adverse reactions such as myalgia, myositis, liver function damage and rhabdomyolysis in clinical application, and the risk of new onset of diabetes is increased by taking statins for a long time, with the incidence rate being about 10-12%, belonging to the statins effect; ezetimibe, a cholesterol absorption inhibitor, is mainly manifested by headache and symptoms in the digestive tract, and can also have side effects such as transaminase increase and myalgia and be weak in cholesterol reducing effect when used in combination with statins. With the long-term use of statins, drug tolerance has emerged in a large number of patients. Statistics show that 60% of high-risk patients still cannot sufficiently control LDL-C level by taking statins or other lipid-lowering drugs on the market, and the proportion in the high-risk patients is up to more than 80%. Particularly for patients having heterozygous familial hypercholesterolemia (HeFH) or homozygous familial hypercholesterolemia (HoFH) with a high risk of cardiovascular diseases, patients with LDL-C level which is not up to standard who have statin intolerance or have been subjected to treatment by using first-line or second-line lipid-lowering drugs at the highest tolerance dose still have an urgent clinical need of a cholesterollowering drug based on a new mechanism.

PCSK9 (proprotein convertase subtilin/kexin type 9) is the 9^{th} member of the proprotein convertase family. PCSK9 is expressed primarily in the liver, but is also detectable in other organs, including the intestine, kidney, pancreas and brain. PCSK9 secreted by liver into circulation is combined with LDLR on the surface of liver cells, and the formed compound is swallowed and promotes degradation of LDLR in lysosome, thereby preventing LDLR from being recirculated to cell membranes, reducing reutilization of LDLR, reducing level of LDLR in liver cell membranes, and causing elimination and reduction of LDL-C. PCSK9 plays a key role in cholesterol balance by lowering the concentration of LDLR on liver surface. Aiming at the mechanism, a class of new drugs PCSK9 monoclonal antibodies are recently developed, which can reduce the circulating level of PCSK9, increase the expression of LDLR on cell surface, and increase the clearance of LDL-C, and thus reduce the circulating level of LDL-C. From the mechanism of action, these drugs can be effective in reducing LDL-C levels in all patients who express LDLR on the liver surface. Such drugs are therefore effective in a large proportion of patients, particularly patients who are statin intolerant or who have failed to effectively control LDL-C levels after having been subjected to statin treatment at a maximum dose.

PCSK9 monoclonal antibodies currently marketed are mainly Evolocumab (Repatha) developed by Amgen and Alirocumab (Praluent) developed by Sanofi and Regeneron in combination. Two marketed PCSK9 monoclonal antibodies reduce LDL-C by about 50%-60% when used in combination with statins. A large number of experiments have demonstrated the safety, good tolerability and efficacy of the PCSK9 monoclonal antibodies. Clinical trial subjects include subjects having primary hypercholesterolemia (including heterozygous familial and non-familial hypercholesterolemia) and mixed hypercholesterolemia, including patients with substandard LDL-C level who are unable to tolerate statin treatment, statins, and/or other lipid-lowering drugs to lower LDL-C level; in addition, clinical trials are also carried out on patients having homozygous familial hypercholesterolemia, and certain therapeutic effects are achieved. The administration regimen for Evolocumab is 140 mg Q2W or 420mg Q4W. The recommended dose of the administration regimen for Alirocumab is 75mg Q2W, with an optional starting dose also comprising 300 mg Q4W and a variable maximum dose of 150 mg Q2W. WO2017114230A, WO2017118307A, WO2018228406 and WO2019001560 disclose new PCSK9 antibodies other than Evolocumab and Alirocumab.

### SUMMARY

The present disclosure provides a method for lowering a serum LDL cholesterol level in a patient, or for treating and/or preventing hyperlipidemia in a patient, or for treating and/or preventing cholesterol-related diseases, which has a simple scheme, low administration frequency and small administration dosage, and can effectively reduce the treatment burden of the patient and improve the treatment compliance of the patient.

In one aspect, the present disclosure provides a method for lowering a serum LDL cholesterol level in a patient, or for treating and/or preventing hyperlipidemia in a patient, or for treating and/or preventing cholesterol-related diseases, comprising administering to a patient at least one PCSK9 antibody or an antigen-binding fragment thereof at a dose of up to 600 mg each time and an administration interval of not shorter than 4 weeks, wherein the PCSK9 antibody or the antigen-binding fragment thereof has LCDR1, LCDR2 and LCDR3 as set forth in SEQ ID NO: 4, SEQ ID NO: 5 and SEQ ID NO: 6, respectively, and HCDR1, HCDR2 and HCDR3 as set forth in SEQ ID NO: 1, SEQ ID NO: 2 and SEQ ID NO: 3, respectively, and the administration interval is preferably 4, 5, 6, 7, 8, 9, 10, 11, 12, 13, 14, 15 or 16 weeks.

Among them, the CDR sequences are set forth in the following table:

| Name | Sequence | No. |
|---|---|---|
| HCDR1 | DYWMH | SEQ ID NO: 1 |
| HCDR2 | YINPSSGFTKYHQNFKD | SEQ ID NO: 2 |
| HCDR3 | QYDYDEDWYFDV | SEQ ID NO: 3 |
| LCDR1 | KSSQSLLNSRTRKNFLA | SEQ ID NO: 4 |
| LCDR2 | WASTRES | SEQ ID NO: 5 |
| LCDR3 | KQSFNLFT | SEQ ID NO: 6 |

In embodiments of the present disclosure, an amount for lowering the serum LDL cholesterol (LDL-C) level in a patient treated with the method of the present disclosure is selected from the group consisting of: a) at least 15%, b) at least 20%, c) at least 30%, d) at least 40%, e) at least 50%, f) at least 55%, g) at least 60%, h) at least 65%, i) at least 70%, j) at least 75%, k) at least 80%, l) at least 85%, and m) at least 90%. In some embodiments, the lowering in the serum LDL cholesterol level in a patient treated with the method of the present disclosure may last for 7-84 days, e.g., 7-14 days, 14-21 days, 21-28 days, 28-35 days, 35-42 days, 42-49 days, 49-56 days, 56-63 days, 63-70 days, 70-77 days, or 77-84 days.

In alternative embodiments, the PCSK9 antibody or the antigen-binding fragment thereof is a recombinant antibody or an antigen-binding fragment thereof selected from the group consisting of a murine antibody, a chimeric antibody and a humanized antibody.

In alternative embodiments, the sequences of the light and heavy chain framework regions on the light and heavy chain variable regions of the humanized antibody are sequences or mutated sequences thereof of human light and heavy chain framework regions.

In alternative embodiments, the PCSK9 antibody or the antigen-binding fragment thereof has a light chain variable region as set forth in SEQ ID NO: 8 and a heavy chain variable region as set forth in SEQ ID NO: 7, respectively.

### Heavy chain variable region

QVQLVQSGAEVKKPGASVKVSCKASGYTFTDYWMHWVRQAPGQGLEWMGYINPSSGFT
KYHQNFKDRVTMTRDTSISTAYMELSRLRSDDTAVYYCARQYDYDEDWYFDVWGQGTTV
TVSS
SEQ ID NO: 7

### Light chain variable region

DIQMTQSPSSLSASVGDRVTITCKSSQSLLNSRTRKNFLAWYQQKPGKAPKLLIYWASTR
ESGVPSRFSGSGSGTDFTLTISSLQPEDFATYYCKQSFNLFTFGQGTKLEIK
SEQ ID NO: 8

In some alternative embodiments, the PCSK9 antibody or the antigen-binding fragment thereof comprises a heavy chain constant region of human IgG1, IgG2, IgG3 or IgG4 isotype, and preferably comprises a heavy chain constant region of IgG1 isotype.

In other alternative embodiments, the PCSK9 antibody or the antigen-binding fragment thereof comprises a light chain constant region that is a κ or λ light chain constant region.

In some alternative embodiments, the PCSK9 antibody or the antigen-binding fragment thereof comprises a light chain having a sequence set forth in SEQ ID NO: 10 and a heavy chain having a sequence set forth in SEQ ID NO: 9.

In alternative embodiments, the PCSK9 antibody is an h001-4-YTE antibody. The h001-4-YTE antibody has a light chain sequence as set forth in SEQ ID NO: 10 and a heavy chain sequence as set forth in SEQ ID NO: 9.

### Heavy chain

QVQLVQSGAEVKKPGASVKVSCKASGYTFTDYWMHWVRQAPGQGLEWMGYINPSS
GFTKYHQNFKDRVTMTRDTSISTAYMELSRLRSDDTAVYYCARQYDYDEDWYFDVW
GQGTTVTVSSASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKKVEPKSCDKTHT
CPPCPAPELLGGPSVFLFPPKPKDTLYITREPEVTCVVVDVSHEDPEVKFNWYVDGVEV
HNAKTKPREEQYNSTYRVVSVLTVLHQDWLNGKEYKCKVSNKALPAPIEKTISKAKG
QPREPQVYTLPPSRDELTKNQVSLTCLVKGFYPSDIAVEWESNGQPENNYKTTPPVLDS
DGSFFLYSKLTVDKSRWQQGNVFSCSVMHEALHNHYTQKSLSLSPGK
SEQ ID NO: 9

### Light chain

DIVMSQSPSSLSASVGDRVTITCKSSQSLLNSRTRKNFLAWYQQKPGKSPKLLIYWAST
RESGVPDRFSGSGSGTDFTLTISSLQPEDFATYYCKQSFNLFTFGQGTKLEIKRTVAAPSV
FIFPPSDEQLKSGTASVVCLLNNFYPREAKVQWKVDNALQSGNSQESVTEQDSKDSTY
SLSSTLTLSKADYEKHKVYACEVTHQGLSSPVTKSFNRGEC
SEQ ID NO: 10

The content of WO2019001560A is incorporated herein by reference in its entirety.

In alternative embodiments, the PCSK9 antibody or the antigen-binding fragment thereof is administered at a dose selected from the group consisting of 70-200 mg, e.g., 70-150 mg; the administration interval is selected from the group consisting of 4, 5, 6, 7, 8, 9 and 10 weeks, e.g., 4 or 8 weeks.

In alternative embodiments, the PCSK9 antibody or the antigen-binding fragment thereof is administered at a dose selected from the group consisting of 100-300 mg, e.g., 150-300 mg; the administration interval is not shorter than 6 weeks, e.g., 6, 7, 8, 9, 10, 11, 12, 13 or 14 weeks, e.g., 8 or 12 weeks.

In alternative embodiments, the PCSK9 antibody or the antigen-binding fragment thereof is administered at a dose selected from the group consisting of 250-550 mg, e.g., 300-450 mg; the administration interval is not shorter than 8 weeks, e.g., 8, 9, 10, 11, 12, 13, 14, 15 or 16 weeks, e.g., 8 or 12 weeks.

In alternative embodiments, the PCSK9 antibody or the antigen-binding fragment thereof is administered at a dose selected from the group consisting of: a) 60-550 mg, b) 70 mg, c) 75 mg, d) 100 mg, e) 120 mg, f) 130 mg, g) 140 mg, h) 150 mg, i) 210 mg, j) 280 mg, k) 300 mg, l) 350 mg, m) 400 mg, n) 450 mg, o) 500 mg, and p) 550 mg.

In alternative embodiments, the PCSK9 antibody or the antigen-binding fragment thereof is administered at an interval selected from the group consisting of 1, 2, 4, 8, 12 and 16 weeks.

In alternative embodiments, the administration regimen for the PCSK9 antibody or the antigen-binding fragment thereof is selected from the group consisting of 75 mg Q4W, 150 mg Q8W, 300 mg Q12W, 150 mg Q4W, 300 mg Q8W and 450 mg Q12W.

In some embodiments of the present disclosure, the PCSK9 antibody or the antigen-binding fragment thereof is administered parenterally. In some embodiments of the present disclosure, the PCSK9 antibody or the antigen-binding fragment thereof is administered intravenously. In some embodiments of the present disclosure, the PCSK9 antibody or the antigen-binding fragment thereof is administered subcutaneously. For example, an injectable form of the anti-PCSK9 antibody or the antigen-binding fragment thereof is an injection solution or a lyophilized powder for injection comprising the anti-PCSK9 antibody or the antigen-binding fragment thereof, a buffer and a stabilizer, and optionally further comprising a surfactant. The buffer is a histidine-hydrochloride system; the stabilizer may be selected from the group consisting of sugars and amino acids, e.g., disaccharides, such as sucrose, lactose, trehalose or maltose. The surfactant is selected from the group consisting of polyoxyethylene hydrogenated castor oil, glycerin fatty acid ester and polyoxyethylene sorbitan fatty acid ester, for example, the polyoxyethylene sorbitan fatty acid ester is polysorbate 20, 40, 60 or 80, such as polysorbate 20. For example, an injectable form of the PCSK9 antibody or the antigen-binding fragment thereof comprises the anti-PCSK9 antibody or the antigen-binding fragment thereof, histidine-hydrochloride buffer, sucrose and polysorbate 80.

In alternative embodiments, the anti-PCSK9 antibody or the antigen-binding fragment thereof is administered subcutaneously to a patient once every two weeks at a dose of 30 mg to 70 mg, wherein the serum LDL cholesterol level of the patient is lowered by at least 15% to at least 50% for 7 to 14 days; the anti-PCSK9 antibody or the antigen-binding fragment thereof is administered subcutaneously to a patient once every two weeks at a dose of 30 mg, wherein the serum LDL cholesterol level of the patient is lowered by at least 15% to at least 50% for 7 to 14 days; the anti-PCSK9 antibody or the antigen-binding fragment thereof is administered subcutaneously to a patient once every two weeks at a dose of 35 mg, wherein the serum LDL cholesterol level of the patient is lowered by at least 15% to at least 50% for 7 to 14 days; the anti-PCSK9 antibody or the antigen-binding fragment thereof is administered subcutaneously to a patient once every two weeks at a dose of 70 mg, wherein the serum LDL cholesterol level of the patient is lowered by at least 15% to at least 50% for 7 to 14 days; the anti-PCSK9 antibody or the antigen-binding fragment thereof is administered subcutaneously to a patient once every four weeks at a dose of 50 mg to 280 mg, wherein the serum LDL cholesterol level of the patient is lowered by at least 15% to at least 50% for 21 to 31 days; the anti-PCSK9 antibody or the antigen-binding fragment thereof is administered subcutaneously to a patient once every four weeks at a dose of 75 mg, wherein the serum LDL cholesterol level of the patient is lowered by at least 15% to at least 50% for 21 to 31 days; the anti-PCSK9 antibody or the antigen-binding fragment thereof is administered subcutaneously to a patient once every four weeks at a dose of 105 mg, wherein the serum LDL cholesterol level of the patient is lowered by at least 15% to at least 50% for 21 to 31 days; the anti-PCSK9 antibody or the antigen-binding fragment thereof is administered subcutaneously to a patient once every four weeks at a dose of 120 mg, wherein the serum LDL cholesterol level of the patient is lowered by at least 15% to at least 50% for 21 to 31 days; the anti-PCSK9 antibody or the antigen-binding fragment thereof is administered subcutaneously to a patient once every four weeks at a dose of 150 mg, wherein the serum LDL cholesterol level of the patient is lowered by at least 15% to at least 50% for 21 to 31 days; the anti-PCSK9 antibody or the antigen-binding fragment thereof is administered subcutaneously to a patient once every four weeks at a dose of 210 mg, wherein the serum LDL cholesterol level of the patient is lowered by at least 15% to at least 50% for 21 to 31 days; the anti-PCSK9 antibody or the antigen-binding fragment thereof is administered subcutaneously to a patient once every four weeks at a dose of 280 mg, wherein the serum LDL cholesterol level of the patient is lowered by at least 15% to at least 50% for 21 to 31 days; the anti-PCSK9 antibody or the antigen-binding fragment thereof is administered subcutaneously to a patient at a dose of 100 mg to 500 mg once every eight weeks, wherein the serum LDL cholesterol level of the patient is lowered by at least 15% to at least 50% for 50 to 56 days; the anti-PCSK9 antibody or the antigen-binding fragment thereof is administered subcutaneously to a patient at a dose of 150 mg once every eight weeks, wherein the serum LDL cholesterol level of the patient is lowered by at least 15% to at least 50% for 50 to 56 days; the anti-PCSK9 antibody or the antigen-binding fragment thereof is administered subcutaneously to a patient at a dose of 300 mg once every eight weeks, wherein the serum LDL cholesterol level of the patient is lowered by at least 15% to at least 50% for 50 to 56 days; the anti-PCSK9 antibody or the antigen-binding fragment thereof is administered subcutaneously to a patient at a dose of 450 mg once every eight weeks, wherein the serum LDL cholesterol level of the patient is lowered by 15% to at least 50% for 50 to 56 days; the anti-PCSK9 antibody or the antigen-binding fragment thereof is administered subcutaneously to a patient at a dose of 150 mg to 500 mg once every twelve weeks, wherein the serum LDL cholesterol level of the patient is lowered by at least 15% to at least 50% for 80 to 84 days; the anti-PCSK9 antibody or the antigen-binding fragment thereof is administered subcutaneously to a patient at a dose of 300 mg once every twelve weeks, wherein the serum LDL cholesterol level of the patient is lowered by at least 15% to at least 50% for 80 to 84 days; the anti-PCSK9 antibody or the antigen-binding fragment thereof is administered subcutaneously to a patient at a dose of 450 mg once every twelve weeks, wherein the serum LDL cholesterol level of the patient is lowered by at least 15% to at least 50% for 80 to 84 days.

In alternative embodiments, the anti-PCSK9 antibody or the antigen-binding fragment thereof is administered subcutaneously to a patient once every two weeks at a dose of 30 mg to 70 mg, wherein the serum LDL cholesterol level of the patient is lowered by at least 30% to at least 50% for 7 to 14 days; the anti-PCSK9 antibody or the antigen-binding fragment thereof is administered subcutaneously to a patient once every two weeks at a dose of 30 mg, wherein the serum LDL cholesterol level of the patient is lowered by at least 30% to at least 50% for 7 to 14 days; the anti-PCSK9 antibody or the antigen-binding fragment thereof is administered subcutaneously to a patient once every two weeks at a dose of 35 mg, wherein the serum LDL cholesterol level of the patient is lowered by at least 30% to at least 50% for 7 to 14 days; the anti-PCSK9 antibody or the antigen-binding fragment thereof is administered subcutaneously to a patient once every two weeks at a dose of 70 mg, wherein the serum LDL cholesterol level of the patient is lowered by at least 30% to at least 50% for 7 to 14 days; the anti-PCSK9 antibody or the antigen-binding fragment thereof is administered subcutaneously to a patient once every four weeks at a dose of 50 mg to 280 mg, wherein the serum LDL cholesterol level of the patient is lowered by at least 30% to at least 50% for 21 to 31 days; the anti-PCSK9 antibody or the antigen-binding fragment thereof is administered subcutaneously to a patient once every four weeks at a dose of 75 mg, wherein the serum LDL cholesterol level of the patient is lowered by at least 30% to at least 50% for 21 to 31 days; the anti-PCSK9 antibody or the antigen-binding fragment thereof is administered subcutaneously to a patient once every four weeks at a dose of 105 mg, wherein the serum LDL cholesterol level of the patient is lowered by at least 30% to at least 50% for 21 to 31 days; the anti-PCSK9 antibody or the antigen-binding fragment thereof is administered subcutaneously to a patient once every four weeks at a dose of 120 mg, wherein the serum LDL cholesterol level of the patient is lowered by at least 30% to at least 50% for 21 to 31 days; the anti-PCSK9 antibody or the antigen-binding fragment thereof is administered subcutaneously to a patient once every four weeks at a dose of 300 mg, wherein the serum LDL cholesterol level of the patient is lowered by at least 30% to at least 50% for 21 to 31 days; the anti-PCSK9 antibody or the antigen-binding fragment thereof is administered subcutaneously to a patient once every four weeks at a dose of 210 mg, wherein the serum LDL cholesterol level of the patient is lowered by at least 30% to at least 50% for 21 to 31 days; the anti-PCSK9 antibody or the antigen-binding fragment thereof is administered subcutaneously to a patient once every four weeks at a dose of 280 mg, wherein the serum LDL cholesterol level of the patient is lowered by at least 30% to at least 50% for 21 to 31 days; the anti-PCSK9 antibody or the antigen-binding fragment thereof is administered subcutaneously to a patient at a dose of 100 mg to 500 mg once every eight weeks, wherein the serum LDL cholesterol level of the patient is lowered by at least 30% to at least 50% for 50 to 56 days; the anti-PCSK9 antibody or the antigen-binding fragment thereof is administered subcutaneously to a patient at a dose of 300 mg once every eight weeks, wherein the serum LDL cholesterol level of the patient is lowered by at least 30% to at least 50% for 50 to 56 days; the anti-PCSK9 antibody or the antigen-binding fragment thereof is administered subcutaneously to a patient at a dose of 300 mg once every eight weeks, wherein the serum LDL cholesterol level of the patient is lowered by at least 30% to at least 50% for 50 to 56 days; the anti-PCSK9 antibody or the antigen-binding fragment thereof is administered subcutaneously to a patient at a dose of 450 mg once every eight weeks, wherein the serum LDL cholesterol level of the patient is lowered by at least 30% to at least 50% for 50 to 56 days; the anti-PCSK9 antibody or the antigen-binding fragment thereof is administered subcutaneously to a patient at a dose of 300 mg to 500mg once every twelve weeks, wherein the serum LDL cholesterol level of the patient is lowered by 30% to at least 50% for 80 to 84 days; the anti-PCSK9 antibody or the antigen-binding fragment thereof is administered subcutaneously to a patient at a dose of 300 mg once every twelve weeks, wherein the serum LDL cholesterol level of the patient is lowered by at least 30% to at least 50% for 80 to 84 days; the anti-PCSK9 antibody or the antigen-binding fragment thereof is administered subcutaneously to a patient at a dose of 450 mg once every twelve weeks, wherein the serum LDL cholesterol level of the patient is lowered by at least 30% to at least 50% for 80 to 84 days.

In alternative embodiments, the PCSK9 antibody or the antigen-binding fragment thereof is administered to a patient with a cholesterol-related disease according to an administration regimen selected from the group consisting of 75 mg Q4W, 150 mg Q8W, 300 mg Q12W, 150 mg Q4W, 300 mg Q8W and 450 mg Q12W, which can be effective to treat the cholesterol-related diseases and lower the serum LDL cholesterol level in the patient. The amount for lowering the serum LDL cholesterol level in a patient is selected from the group consisting of: a) at least 15%, b) at least 30%, c) at least 40%, d) at least 50%, and e) at least 60%.

In alternative embodiments, the PCSK9 antibody or the antigen-binding fragment thereof is administered to a patient in need thereof according to an administration regimen selected from the group consisting of 75 mg Q4W, 150 mg Q8W, 300 mg Q12W, 150 mg Q4W, 300 mg Q8W, and 450 mg Q12W, which is effective to lower the serum LDL cholesterol level in the patient in need thereof. The amount for lowering the serum LDL cholesterol level in a patient is selected from the group consisting of: a) at least 15%, b) at least 30%, c) at least 40%, d) at least 50%, and e) at least 60%.

In some embodiments, the patient has been previously treated with at least one cholesterol synthesis inhibitor and/or cholesterol absorption inhibitor. In some embodiments, the patient has been previously treated with at least one cholesterol synthesis inhibitor or cholesterol absorption inhibitor for at least 4 weeks.

In some embodiments, the cholesterol synthesis inhibitor is a statin. In some embodiments, the cholesterol absorption inhibitor is ezetimibe.

In alternative embodiments of the present disclosure, the at least one anti-PCSK9 antibody is administered to a patient before, after or concurrently with the administration of at least one statin. Statins include, but are not limited to, atorvastatin, cerivastatin, fluvastatin, lovastatin, mevastatin, pitavastatin, pravastatin, rosuvastatin and simvastatin.

In the present disclosure, hyperlipidemia refers to a disease or condition in which abnormally high levels of lipids are present in the blood. Hyperlipidemia may occur in a form selected from the group consisting of: (1) hypercholesterolemia, i.e., elevated cholesterol level; (2) hypertriglyceridemia, i.e., elevated triglyceride level; and (3) combined hyperlipidemia, i.e., a combination of hypercholesterolemia and hypertriglyceridemia.

In the present disclosure, the meaning of hyperlipidemia also extends to hyperlipidemia-related diseases such as atherosclerosis, angina pectoris, carotid artery disease, cerebral arteriosclerosis, xanthoma, coronary heart disease, ischemic stroke, restenosis following angioplasty, peripheral vascular disease, intermittent claudication, decreased necrosis following myocardial infarction, dyslipidemia, postprandial lipemia and pancreatitis.

Hyperlipidemia can be further classified into primary hyperlipidemia and secondary hyperlipidemia. Exemplary primary hyperlipidemias include, but are not limited to: 1) familial hyperchylomicronemia; 2) familial hypercholesterolemia; 3) familial combined hyperlipidemia; 4) familial apolipoprotein B-100 deficiency; 5) familial dysbetalipoproteinemia; and 6) familial hypertriglyceridemia.

Exemplary secondary hyperlipidemias have risk factors including, but not limited to: 1) disease risk factors such as type 1 diabetes, type 2 diabetes, Cushing's syndrome, hypothyroidism, cholestasis and some types of history of renal failure; 2) drug risk factors including contraceptive pills, hormones such as estrogen and corticosteroids, certain diuretics, and various β-blockers; and 3) dietary risk factors including food fat intake of greater than 40% of total calories, a ratio of saturated fat intake to total calories of greater than 10%, a daily cholesterol intake of greater than 300 mg, habitual and excessive alcohol consumption, bulimia, anorexia nervosa and obesity. In the present disclosure, the cholesterol-related diseases include, but are not limited to, hypercholesterolemia, heart disease, metabolic syndrome, diabetes, coronary heart disease, stroke, cardiovascular disease, Alzheimer's disease and dyslipidemia in general.

In some embodiments, the cholesterol-related disease is hypercholesterolemia. In some embodiments, the hypercholesterolemia is selected from the group consisting of primary hypercholesterolemia and secondary hypercholesterolemia. In some embodiments, the primary hypercholesterolemia is selected from the group consisting of heterozygous familial hypercholesterolemia and homozygous familial hypercholesterolemia.

In alternative embodiments, administration of the PCSK9 antibody or the antigen-binding fragment thereof to a hyperlipidemia patient undergoing statin-stable treatment can be effective in lowering the serum LDL cholesterol level in the patient in need thereof. The amount for lowering the serum LDL cholesterol level in a patient is selected from the group consisting of: a) at least 15%, b) at least 30%, c) at least 40%, d) at least 50%, and e) at least 60%.

The present disclosure provides a method for lowering a serum LDL cholesterol level in a patient, or for treating and/or preventing hyperlipidemia, or for treating and/or preventing cholesterol-related diseases, comprising administering to a patient at least one anti-PCSK9 antibody or an antigen-binding fragment thereof at a dose of no more than 300 mg each time and an administration interval of not shorter than 4 weeks, e.g., 4, 5, 6, 7, 8, 9, 10, 11, 12, 13, 14, 15 or 16 weeks.

In some embodiments, the dose administered is 70-250 mg, e.g., 75-150 mg; and/or the administration interval is selected from the group consisting of 4 weeks, 8 weeks, 12 weeks or 16 weeks, e.g., 4 weeks.

In some embodiments, the anti-PCSK9 antibody or the antigen-binding fragment thereof is administered at a dose selected from the group consisting of: a) 75 mg, b) 100 mg, c) 150 mg, d) 200 mg, and e) 250 mg, e.g., 150 mg.

In some embodiments, the anti-PCSK9 antibody or the antigen-binding fragment thereof is administered according to an administration regimen selected from the group consisting of 75 mg Q4W, 150 mg Q4W and 150 mg Q8W.

The present disclosure provides a method for lowering a serum LDL cholesterol level in a patient, or for treating and/or preventing hyperlipidemia in a patient, or for treating and/or preventing cholesterol-related diseases, comprising administering to a patient at least one anti-PCSK9 antibody or an antigen-binding fragment thereof at a dose of 300-600 mg each time and an administration interval of greater than 4 weeks.

In alternative embodiments, the dose administered is selected from the group consisting of 300-500 mg, such as 300 mg or 450 mg.

In alternative embodiments, the administration interval is selected from the group consisting of 5, 6, 7, 8, 9, 10, 11, 12, 13, 14, 15 and 16 weeks.

In alternative embodiments, the administration interval is selected from the group consisting of 8, 12 and 16 weeks.

In some embodiments, the anti-PCSK9 antibody or the antigen-binding fragment thereof is administered according to an administration regimen selected from the group consisting of 300 mg Q8W, 300 mg Q12W and 450 mg Q12W.

In some embodiments, the treatment cycle is at least 4 weeks, at least 6 weeks, at least 8 weeks, at least 10 weeks, at least 12 weeks, at least 16 weeks, at least 20 weeks, at least 24 weeks, at least 28 weeks, at least 32 weeks, at least 36 weeks, at least 40 weeks, at least 44 weeks, at least 48 weeks, at least 52 weeks, at least 56 weeks, at least 1 year, at least 2 years, or longer.

### BRIEF DESCRIPTION OF THE DRAWINGS

FIG. 1 shows the mean percentage change in LDL-C concentration throughout the treatment period.
FIG. 2 shows the mean percentage change in TC concentration throughout the treatment period.
FIG. 3 shows the mean percentage change in non-HDL-C concentration throughout the treatment period.
FIG. 4 shows the mean percentage change in ApoB concentration throughout the treatment period.
FIG. 5 shows the mean percentage change in Lp(a) concentration throughout the treatment period.
FIG. 6 shows the mean percentage change in free PCSK9 concentration throughout the treatment period.

### DETAILED DESCRIPTION

### 1. Terminology

The three-letter and single-letter codes for amino acids used in the present disclosure are described as in J. Biol. Chem, 243, p3558 (1968).

The term "antibody" described in the present disclosure refers to an immunoglobulin, which is of a tetrapeptide chain structure formed by connection between two identical heavy chains and two identical light chains by interchain disulfide bonds.

In the present disclosure, the antibody light chain of the present disclosure may further comprise a light chain constant region comprising human or murine κ and λ chains or variants thereof.

In the present disclosure, the antibody heavy chains of the present disclosure may further comprise a heavy chain constant region comprising human or murine IgG1, IgG2, IgG3 and IgG4 or variants thereof.

In the heavy and light chains of antibody, the sequences of about 110 amino acids near the N-terminus vary considerably and thus are referred to as variable regions (Fv regions); the remaining amino acid sequences near the C-terminus are relatively stable and thus are referred to as constant regions. The variable regions comprise 3 hypervariable regions (HVRs) and 4 framework regions (FRs) with relatively conservative sequences. The 3 hypervariable regions determine the specificity of the antibody and thus are also known as complementarity determining regions (CDRs). Each light chain variable region (LCVR) or heavy chain variable region (HCVR) consists of 3 CDRs and 4 FRs arranged from the amino-terminus to the carboxyterminus in the following order: FR1, CDR1, FR2, CDR2, FR3, CDR3 and FR4. The 3 CDRs of the light chain refer to LCDR1, LCDR2 and LCDR3, and the 3 CDRs of the heavy chain refer to HCDR1, HCDR2 and HCDR3. The CDR amino acid residues of the LCVRs and HCVRs of the antibodies or antigen-binding fragments described in the present disclosure correspond with known Kabat numbering scheme (LCDRs 1-3, HCDRs 1-3) in terms of number and positions. The antibody of the present disclosure includes a murine antibody, a chimeric antibody and a humanized antibody, and preferably a humanized antibody.

The term "murine antibody" used in the present disclosure refers to a monoclonal antibody to human PCSK9 prepared according to the knowledge and skill in the art. During the preparation, a test subject is injected with a PCSK9 antigen, and then hybridoma of antibodies expressing the desired sequence or functional properties is isolated.

The term "chimeric antibody" refers to an antibody obtained by fusing a variable region of a murine antibody and a constant region of a human antibody, which can reduce an immune response induced by the murine antibody. The chimeric antibody is established by firstly establishing hybridoma secreting murine specific monoclonal antibody, then cloning a variable region gene from the mouse hybridoma cells, cloning a constant region gene of human antibody as required, connecting the mouse variable region gene and the human constant region gene into a chimeric gene, inserting the chimeric gene into an expression vector, and finally expressing chimeric antibody molecules in a eukaryotic system or prokaryotic system. In an alternative embodiment of the present disclosure, the antibody light chain of the PCSK9 chimeric antibody further comprises a light chain constant region of human κ and λ chains or variants thereof. The antibody heavy chain of the PCSK9 chimeric antibody further comprises a heavy chain constant region of human IgG1, IgG2, IgG3 and IgG4 or variants thereof. The constant region of the human antibody may be selected from the group consisting of the heavy chain constant regions of human IgG1, IgG2, IgG3 and IgG4 or variants thereof, for example comprising human IgG2 or IgG4 heavy chain constant regions, or IgG4 mutated at amino acids without ADCC (antibodydependent cell-mediated cytotoxicity) toxicity.

The term "humanized antibody", also known as a CDR-grafted antibody, refers to an antibody produced by grafting murine CDR sequences into a human antibody variable region framework, i.e., a different type of human antibody framework sequence. Therefore, the strong antibody variable antibody reaction induced by a large amount of mouse protein components contained in the chimeric antibody can be overcome. Such framework sequences can be obtained from public DNA databases or published references that include germline antibody gene sequences. For example, germline DNA sequences of genes of the human heavy and light chain variable regions can be found in the "VBase" human germline sequence database (available at the Internet address www.mrccpe.com.ac.uk/vbase), as well as in Kabat, E. A. et al., 1991 Sequences of Proteins of Immunological Interest, 5th edition. To avoid decreased immunogenicity and decreased activity at the same time, the human antibody variable region framework sequences may be minimally reversely-mutated or back-mutated to retain activity. The humanized antibody of the present disclosure also includes the humanized antibody formed after further affinity maturation on the CDRs by phage display.

"PCSK9 antibody" is an antibody that specifically binds to PCSK9, including but not limited to the h001 series of PCSK9 humanized antibodies of PCT/CN2016/111053. The h001 series of PCSK9 humanized antibodies are obtained by screening human PCSK9 immunized mice and performing humanized transformation.

"Antigen-binding fragment" described in the present disclosure refers to Fab fragments, Fab' fragments, F(ab')₂ fragments and ScFv fragments that bind to human PCSK9, as well as other fragments that can bind to human PCSK9 using the VH and VL regions of an antibody that can bind to human PCSK9; the antigen-binding fragment comprises one or more CDRs of the antibody of the present disclosure selected from the group consisting of SEQ ID NO: 7 and SEQ ID NO: 8. The Fv fragment contains heavy and light chain variable regions of an antibody, but no constant regions, and has the smallest antibody fragment of the entire antigen-binding site. Generally, Fv antibodies also comprise a polypeptide linker between the VH and VL domains, and are capable of forming the structure required for antigen binding. Two antibody variable regions can also be linked into a single polypeptide chain using different linkers, known as single chain antibody (scFv) or single chain fv (sFv). The term "bind to PCSK9" in the present disclosure refers to the ability to interact with human PCSK9. The term "antigen-binding site" in the present disclosure refers to a continuous or discontinuous three-dimensional spatial site on an antigen that is recognized by the PCSK9 antibody or an antigen-binding fragment.

"Treating" or "treatment" refers to administering a therapeutic agent, such as a composition comprising any one of the antibodies of the present disclosure, either internally or externally to a patient with one or more symptoms of a disease on which the therapeutic agent is known to have a therapeutic effect. Typically, the therapeutic agent is administered in an amount effective to alleviate one or more symptoms of the disease in the patient or population being treated to induce regression of such symptoms or inhibiting the development of such symptoms to any clinically measurable degree. The amount of therapeutic agent effective to alleviate the symptoms of any particular disease (also referred to as a "therapeutically effective amount") may vary depending on a variety of factors, such as the disease state, age, and weight of the patient, and the ability of the drug to produce a desired therapeutic effect in the patient. Whether a symptom of a disease has been alleviated can be evaluated by any clinical testing methods commonly used by doctors or other health care professionals to evaluate the severity or progression of the symptom. Although the embodiments of the present disclosure (for example treatment methods or products) may not be effective in alleviating all the target disease symptoms in every patient, as determined according to any statistical testing methods known in the art, such as Student t-test, chi-square test, Mann and Whitney's U test, Kruskal-Wallis test (H test), Jonckheere-Terpstra test and Wilcoxon test, they should reduce the target disease symptoms in a statistically significant number of patients.

"Effective amount" comprises an amount sufficient to ameliorate or prevent a symptom or sign of a medical condition. An effective amount also refers to an amount sufficient to allow or facilitate diagnosis. The effective amount for a particular patient or veterinary subject may vary depending on the factors such as the condition to be treated, the general health of the patient, the method and route and dosage of administration, and the severity of side effects. An effective amount can be the maximum dose or administration regimen to avoid significant side effects or toxic effects.

Illustratively, "Q4W" indicates administration once every 4 weeks, and "Q8W" indicates administration once every 8 weeks. "150 mg Q8W" indicates a dose of 150 mg each time administered once every 8 weeks.

Familial hyperchylomicronemia is a rare genetic disorder that results in a deficiency of the LP lipase, an enzyme that breaks down fat molecules. The deficiency of LP lipase can lead to a large accumulation of fat or lipoproteins in the blood.

Familial hypercholesterolemia, a relatively common genetic disorder, has the potential defect of a series of mutations in the LDL receptor gene, resulting in dysfunction and/or deficiency of the LDL receptor. This results in inefficient clearance of LDL by LDL receptors, resulting in elevated plasma LDL and total cholesterol levels.

Familial combined hyperlipidemia, also known as combined lipoprotein-type hyperlipidemia, is a genetic disease in which patients and their affected first-degree relatives may exhibit high cholesterol and high triglycerides at different times. HDL cholesterol levels are often moderately reduced.

The deficiency of familial apolipoprotein B-100 is a relatively common autosomal dominant genetic abnormality. This deficiency is caused by a single nucleotide mutation of glutamine in place of arginine, and the mutation results in a decrease in the affinity of LDL particles for LDL receptors. As a result, high plasma LDL and total cholesterol levels are caused.

Familial dysbetalipoproteinemia, also known as type III hyperlipoproteinemia, is a rare genetic disease that results in moderate to severe elevation of serum TG and cholesterol levels and abnormal apolipoprotein E function. HDL levels are often normal.

Familial hypertriglyceridemia is a common genetic disorder with elevated plasma VLDL concentration. This results in a mild to moderate elevation of triglyceride level (cholesterol level is usually not elevated) and is often accompanied by low plasma HDL level.

### Example 1: A Randomized, Double-Blind, Placebo Parallel-Controlled Phase Ib/II Clinical Trial to Study the Safety, Tolerability and Efficacy of Multiple Subcutaneous Injections of h001-4-YTE in Hyperlipidemic Subjects

### 1. Test drug

h001-4-YTE

### 2. Enrolled subject

Subjects must meet all of the following inclusion criteria to qualify for entry into the trial:
(1) subjects who were receiving statin treatments or were eligible for statin treatments at the time of screening, who were receiving statin treatments at a stable dose for more than or equal to 28 days before randomization and who were willing to maintain stable statin treatments in the study (statin species and doses allowed in the study: atorvastatin at 10-40 mg/d) (Lipitor, Pfizer Pharmaceutical Co., Ltd.);
(2) subjects receiving treatments with statin and/or other lipid-regulating drugs at the time of screening who had the serum LDLC of more than or equal to 2.6 mmol/L, or subjects not having received treatments with lipid-regulating drugs at the time of screening who had the serum LDL-C of more than or equal to 3.4 mmol/L and the LDL-C of still more than or equal to 2.6 mmol/L before randomization;
(3) subjects having triglyceride concentration of less than or equal to 4.5 mmol/L during fasting; and
(4) subjects having body mass index (BMI) of more than or equal to 18 kg/m² and less than or equal to 35 kg/m².

### 3. Administration method

In this trial, 6 groups were planned to be divided, including 75 mg Q4W (group 1), 150 mg Q8W (group 2), 300 mg Q12W (group 3), 150 mg Q4W (group 4), 300 mg Q8W (group 5) and 450 mg Q12W (group 6) injected subcutaneously, with Q4W and Q8W regimens administered for 16 weeks and Q12W regimen administered for 24 weeks, respectively.

### 4. Pharmacodynamic data

110 subjects participated in clinical trials and were randomly grouped and administered with H001-4-YTE and placebo at a ratio of 5:1, respectively. All patients received a stable treatment regimen of atorvastatin (10-40 mg/day) for at least 28 days prior to randomization and throughout the study. All doses and time schedules in the H001-4-YTE groups achieved a significant reduction in LDL-C from baseline to the end of the administration interval compared to the placebo group. As can be seen from Table 1, the mean percentage reduction in LS of LDL-C in the H001-4-YTE administration regimens of 75 mg Q4W, 150 mg Q4W, 150 mg Q8W, 300 mg Q8W, 300 mg Q12W and 450 mg Q12W was -48.63% (95% CI: -59.795, -37.455), -55.06% (-62.956, -47.172), -52.02% (-63.188, -40.859), -48.38% (-56.321, -40.440), -43.93% (-55.097, -32.769) and -52.77% (-60.487, -45.054), respectively, while the placebo group had an increase of 4.44% (-3.688, 12.570) (all p values were < 0.0001). The mean percentage reduction in LS of LDL-C in each H001-4-YTE group ranged from -48.33% to -59.00% compared to the placebo group. The mean absolute reduction in LS of LDL-C in each H001-4-YTE group ranged from - 1.59 mmol/L to -1.92 mmol/L compared to the placebo group.

**Table 1. Change in LDL-C from baseline to end of administration interval**

| | **Placebo (N=19)** | **H001-4-YTE** | | | | | |
|---|---|---|---|---|---|---|---|
| | | **75 mg Q4W (N=10)** | **150 mg Q4W (N=20)** | **150 mg Q8W (N=10)** | **300 mg Q8W (N=20)** | **300 mg Q12W (N=10)** | **450 mg Q12W (N=21)** |
| **Baseline (mmol/L), mean (SD)** | 3.360 (0.9283) | 3.656 (0.9510) | 3.563 (0.9001) | 3.464 (0.7534) | 3.759 (0.8739) | 3.608 (0.6738) | 3.420 (0.8351) |
| **Change percentage (%)** | | | | | | | |
| Mean LS (95% Cl) | 4.44 (-3.688,12.570) | -48.63 (-59.795,-37.455) | -55.06 (-62.956,-47.172) | -52.02 (-63.188,-40.859) | -48.38 (-56.321,-40.440) | -43.93 (-55.097,-32.769) | -52.77 (-60.487,-45.054) |
| Compared to change of placebo, mean LS (95% CI); p | --- | -53.07 (-66.906,-39.227); p<0.0001 | -59.51 (-70.841,-48.170); p<0.0001 | -56.46 (-70.259,-42.670); p<0.0001 | -52.82 (-64.241,-41.402); p<0.0001 | -48.37 (-62.198,-34.550); p<0.0001 | -57.21 (-68.389,-46.035); p<0.0001 |
| **Absolute change (mmol/L)** | | | | | | | |
| Mean LS (95% CI) | 0.02 (-0.270,0.313) | -1.69 (-2.091,-1.290) | -1.9 (-2.182,-1.616) | -1.84 (-2.243,-1.443) | -1.73 (⁻2.010,-1.440) | -1.57 (-1.973,-1.173) | -1.85 (-2.129,-1.576) |
| Compared to change of placebo, mean LS (95% CI); p | --- | -1.71 (-2.208,-1.216); p<0.0001 | -1.92 (-2.327,-1.514); p<0.0001 | -1.86 (-2.359,-1.370); p<0.0001 | -1.75 (-2.156,-1.337); p<0.0001 | -1.59 (-2.090,-1.099); p<0.0001 | -1.87 (-2.275,-1.473); p<0.0001 |

| | | | | | | | |
|---|---|---|---|---|---|---|---|
| LS, least-squares method. | | | | | | | |

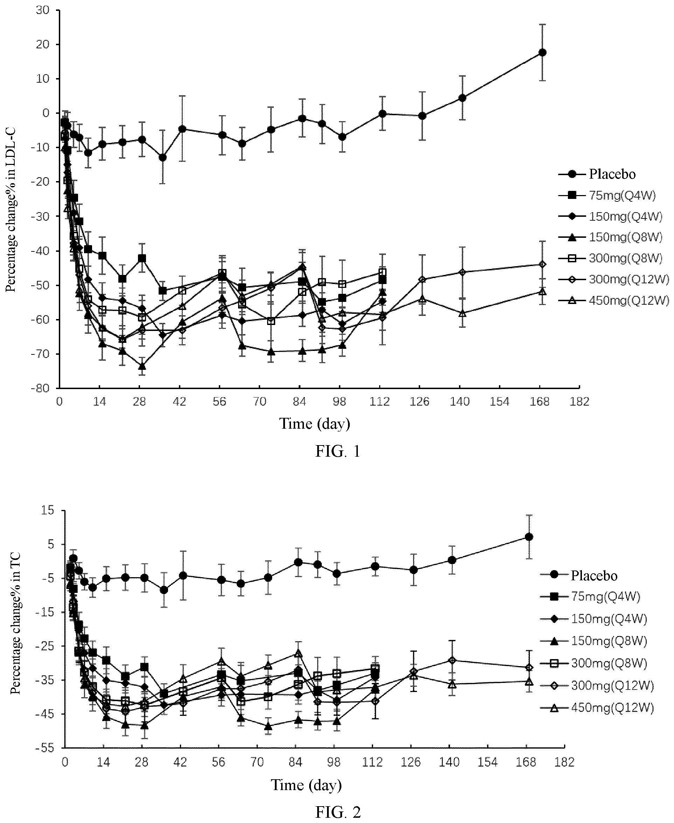

FIG. 1 shows the mean percentage change in LDL-C concentration throughout the treatment period. After the initial administration (week 0), LDL-C in all H001-4-YTE groups decreased rapidly. In the first cycle, the greatest reduction in LDL-C level occurred at week 3 of 75 mg Q4W (-48.182%) and week 4 of 150 mg Q4W (-56.744%); thereafter, the percentage change in LDL-C in the H001-4-YTE 75 mg Q4W group remained between -54.916% and -42.108%, and the percentage change in LDL-C in the H001-4-YTE 150 mg Q4W group remained between - 64.527% and -54.579%. In the first cycle, the maximum reduction in LDL-C occurred at week 4 of 150 mg Q8W (-73.519%) and 300 mg Q8W (-59.274%) and week 3 of 300 mg Q12W (-65.721%) and 450 mg Q12W (-65.522%); thereafter, a small rebound occurred with readministration; however, the strongly decreasing trend still existed, the percentage change in LDL-C in the 150 mg Q8W group ranged from -52.050% to -69.323%, the percentage change in LDL-C in the 300 mg Q8W group ranged from -46.133% to -60.476%, the percentage change in LDL-C in the 300 mg Q12W group ranged from -43.909% to -63.108%, and the percentage change in LDL-C in the 450 mg Q12W group ranged from -44.491% to -59.556%.

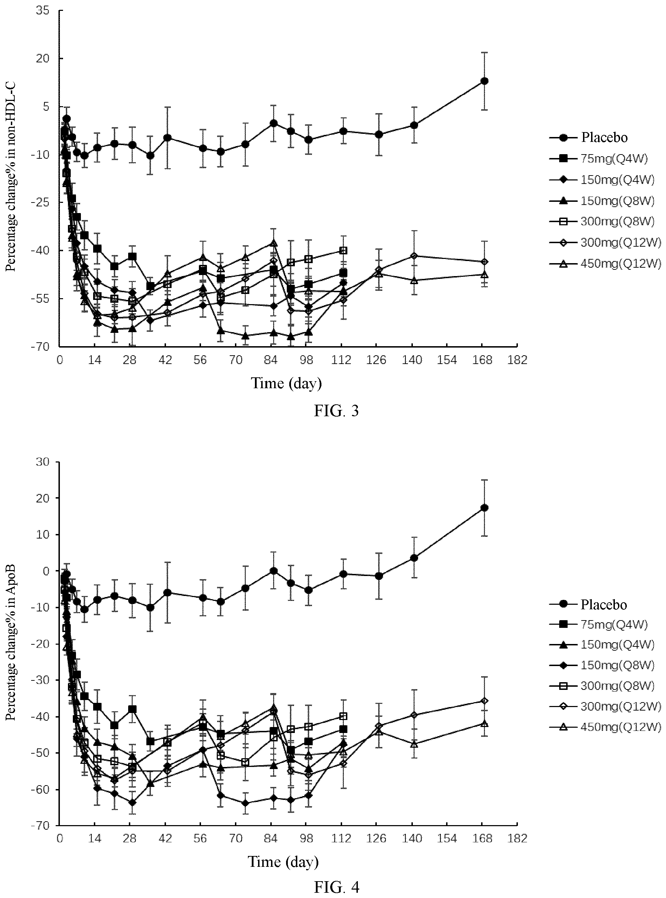

Table 2 summarizes the percentage reduction in other lipid and apolipoprotein measurements. Sustained rapid reductions in TC, non-HDL-C, ApoB and Lp(a) were observed in all H001-4-YTE groups (FIGs. 2-5). The percentage change in TC from baseline to the end of the administration interval ranged from -31.263% to -37.760% in the H001-4-YTE groups and from 3.534% to -3.065% in the placebo group. The percentage change in non-HDL-C from baseline to the end of the administration interval ranged from -39.920% to -52.349% in the H001-4-YTE groups and from 7.412% to -5.957% in the placebo group. The percentage change in ApoB from baseline to the end of the administration interval ranged from -35.592% to -47.687% in the H001-4-YTE groups and from 12.565% to -6.623% in the placebo group. The percentage change in Lp(a) from baseline to the end of the administration interval ranged from -31.263% to -35.339% in the H001-4-YTE groups and from 1.578% to -3.065% in the placebo group.

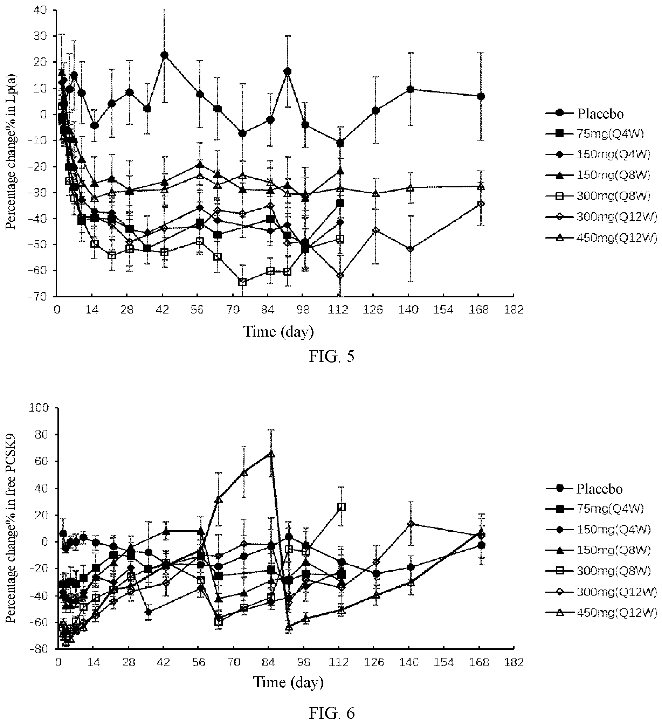

A sharp reduction in free PCSK9 and a dose-dependent percentage reduction were detected immediately after administration and H001-4-YTE treatment (FIG. 6). On day 2, free PCSK9 in the H001-4-YTE 75 mg Q4W group decreased by -31.60% (SE, 7.462%), free PCSK9 in the H001-4-YTE 150 mg Q4W group decreased by -37.09% (5.645%), and free PCSK9 in the placebo Q4W group decreased by -9.44% (11.048%); free PCSK9 in the H001-4-YTE 150 mg Q8W group decreased by -39.12% (5.760%), and free PCSK9 in the H001-4-YTE 300 mg Q8W group decreased by -63.88% (4.079%), while free PCSK9 in the placebo Q8W group increased by 10.17% (8.691%); free PCSK9 in the H001-4-YTE 300 mg Q12W group decreased by - 61.59% (4.507%), and free PCSK9 in the H001-4-YTE 450 mg Q12W group decreased by - 68.56% (4.554%), while free PCSK9 in the placebo Q12W group increased by 16.57% (28.585). In addition, no patients experienced serious treatment emergent adverse events (TEAEs) during the study, no TEAEs resulting in discontinuation of treatment or death, and no injection site reactions reported.

**Table 2. Reduction in LDL-C from baseline to 12 weeks and reduction in lipid and apolipoprotein measurements from baseline to end of administration interval after H001-4-YTE treatment**

| | **Placebo Q4W (N=6)** | **75 mg Q4W (N=10)** | **150 mg Q4W (N=20)** | **Placebo Q8W (N=6)** | **150 mg Q8W (N=10)** | **300 mg Q8W (N=20)** | **Placebo Q12W (N=7)** | **300 mg Q12W (N=10)** | **450 mg Q12W (N=21)** |
|---|---|---|---|---|---|---|---|---|---|
| **LDL-C** | | | | | | | | | |
| Baseline (mmol/L) | 2.857 (0.1782) | 3.656 (0.3007) | 3.563 (0.2013) | 3.253 (0.3488) | 3.464 (0.2382) | 3.759 (0.1954) | 3.883 (0.42) | 3.608 (0.2131) | 3.42 (0.1822) |
| Percentage change from baseline to 12 weeks (%) | -4.792 (10.8103) | -48.924 (5.6803) | -58.607 (3.3079) | -6.143 (8.0979) | -68.975 (3.1973) | -51.936 (6.8753) | 13.32 (6.3183) | -43.909 (6.7883) | -51.817 (3.7655) |

| **TC** | | | | | | | | | |
|---|---|---|---|---|---|---|---|---|---|
| Baseline (mmol/L) | 4.57 (0.1647) | 5.169 (0.3291) | 5.352 (0.1665) | 5.223 (0.3582) | 5.304 (0.2714) | 5.455 (0.1735) | 5.83 (0.4728) | 5.186 (0.2323) | 5.081 (0.1907) |
| Percentage change from baseline to end of administration interval (%) | 1.578 (5.8769) | -32.94 (3.048) | -34.162 (3.2339) | -3.065 (4.8358) | -37.76 (3.4319) | -31.576 (3.5157) | 3.534 (5.0572) | -31.263 (5.0053) | -35.339 (3.1863) |

| **Non-HDL-C** | | | | | | | | | |
|---|---|---|---|---|---|---|---|---|---|
| Baseline (mmol/L) | 3.22 (0.2407) | 4.116 (0.3037) | 4.071 (0.1702) | 4.02 (0.3985) | 4.032 (0.2245) | 4.281 (0.1827) | 4.179 (0.36) | 4.029 (0.2233) | 3.78 (0.1822) |
| Percentage change from baseline to end of administration interval (%) | 2.746 (8.3052) | -46.991 (3.6049) | -49.979 (4.3956) | -5.957 (6.1656) | -52.349 (3.3013) | -39.92 (4.4408) | 7.412 (6.4966) | -43.542 (6.5028) | -47.383 (3.9001) |

| **ApoB** | | | | | | | | | |
|---|---|---|---|---|---|---|---|---|---|
| Baseline (g/L) | 0.908 (0.0654) | 1.151 (0.0695) | 1.112 (0.0495) | 1.115 (0.0946) | 1.094 (0.0589) | 1.194 (0.0525) | 1.191 (0.0853) | 1.106 (0.0579) | 1.072 (0.0459) |
| Percentage change from baseline to end of administration interval (%) | 7.087 (7.9552) | -43.392 (3.719) | -47.026 (4.1798) | -6.623 (6.8391) | -47.687 (3.3131) | -39.937 (4.3967) | 12.565 (6.0401) | -35.592 (6.5036) | -41.838 (3.4896) |

| **Lp(a)** | | | | | | | | | |
|---|---|---|---|---|---|---|---|---|---|
| Baseline (g/L) | 8.2 (3.5501) | 9.859 (2.6033) | 11.285 (2.671) | 5.223 (0.8773) | 5.304 (0.8582) | 5.455 (0.7758) | 5.830 (1.2510) | 5.186 (0.7346) | 5.081 (0.8741) |
| Percentage change from baseline to end of administration interval (%) | 1.578 (13.1411) | -32.940 (9.6386) | -34.162 (12.5249) | -3.065 (11.8452) | -37.760 (10.8527) | -31.576 (14.9158) | 3.534 (12.3876) | -31.263 (15.8281) | -35.339 (14.2495) |

| | | | | | | | | | |
|---|---|---|---|---|---|---|---|---|---|
| LDL-C refers to low-density lipoprotein cholesterol; TC refers to total cholesterol; ApoB refers to apolipoprotein B; non-HDL-C refers to non-high-density lipoprotein cholesterol; Lp(a) refers to lipoprotein a. | | | | | | | | | |

## Claims

1. A method for lowering a serum LDL cholesterol level in a patient, or for treating and/or preventing hyperlipidemia in a patient, or for treating and/or preventing cholesterol-related diseases, comprising administering to a patient at least one PCSK9 antibody or an antigen-binding fragment thereof at a dose of up to 600 mg each time and an administration interval of not shorter than 4 weeks, wherein the PCSK9 antibody or the antigen-binding fragment thereof has LCDR1, LCDR2 and LCDR3 as set forth in SEQ ID NO: 4, SEQ ID NO: 5 and SEQ ID NO: 6, respectively, and HCDR1, HCDR2 and HCDR3 as set forth in SEQ ID NO: 1, SEQ ID NO: 2 and SEQ ID NO: 3, respectively, and the administration interval is preferably 4, 5, 6, 7, 8, 9, 10, 11, 12, 13, 14, 15 or 16 weeks.

2. The method according to claim 1, wherein the PCSK9 antibody or the antigen-binding fragment thereof has a light chain variable region as set forth in SEQ ID NO: 8 and a heavy chain variable region as set forth in SEQ ID NO: 7, respectively.

3. The method according to claim 1, wherein the PCSK9 antibody or the antigen-binding fragment thereof comprises a light chain having a sequence set forth in SEQ ID NO: 10 and a heavy chain having a sequence set forth in SEQ ID NO: 9.

4. The method according to any one of claims 1 to 3, wherein the dose administered is selected from the group consisting of 70-200 mg, and preferably 70-150 mg; the administration interval is selected from the group consisting of 4, 5, 6, 7, 8, 9 and 10 weeks, and preferably 4 or 8 weeks.

5. The method according to any one of claims 1 to 3, wherein the dose administered is selected from the group consisting of 100-300 mg, and preferably 150-300 mg; the administration interval is not shorter than 6 weeks, preferably 6, 7, 8, 9, 10, 11, 12, 13 or 14 weeks, and more preferably 8 or 12 weeks.

6. The method according to any one of claims 1 to 3, wherein the dose administered is selected from the group consisting of 250-550 mg, and preferably 300-550 mg; the administration interval is not shorter than 8 weeks, preferably 8, 9, 10, 11, 12, 13, 14, 15 or 16 weeks, and more preferably 8 or 12 weeks.

7. The method according to any one of claims 1 to 3, wherein the PCSK9 antibody or the antigen-binding fragment thereof is administered at a dose selected from the group consisting of: a) 60-550 mg, b) 70 mg, c) 75 mg, d) 100 mg, e) 120 mg, f) 130 mg, g) 140 mg, h) 150 mg, i) 210 mg, j) 280 mg, k) 300 mg, l) 350 mg, m) 400 mg, n) 450 mg, o) 500 mg, and p) 550 mg.

8. The method according to any one of claims 1 to 3, wherein the PCSK9 antibody or the antigen-binding fragment thereof is administered at an interval selected from the group consisting of 4, 8, 12 and 16 weeks.

9. The method according to any one of claims 1 to 3, wherein the PCSK9 antibody or the antigen-binding fragment thereof is administered according to an administration regimen selected from the group consisting of 75 mg Q4W, 150 mg Q8W, 300 mg Q12W, 150 mg Q4W, 300 mg Q8W and 450 mg Q12W.

10. The method according to any one of claims 1 to 3, wherein an amount for lowering the serum LDL cholesterol level is at least 10%.

11. The method according to any one of claims 1 to 3, wherein the amount for lowering the serum LDL cholesterol level is selected from the group consisting of: a) at least 15%, b) at least 20%, c) at least 30%, d) at least 40%, e) at least 50%, f) at least 55%, g) at least 60%, and h) at least 65%.

12. The method according to any one of claims 1 to 3, wherein the hyperlipidemia is selected from the group consisting of hypercholesterolemia and mixed hyperlipidemia.

13. The method according to any one of claims 1 to 3, wherein the cholesterol-related diseases are primary hypercholesterolemia, and preferably heterozygous familial hypercholesterolemia or homozygous familial hypercholesterolemia.

14. The method according to any one of claims 1 to 3, wherein the patient has been previously treated with at least one cholesterol synthesis inhibitor and/or cholesterol absorption inhibitor; preferably, the cholesterol synthesis inhibitor is a statin, and more preferably atorvastatin, cerivastatin, fluvastatin, lovastatin, mevastatin, pitavastatin, pravastatin, rosuvastatin or simvastatin;
preferably, the cholesterol absorption inhibitor is ezetimibe.

15. The method according to claim 14, wherein the patient has been previously treated with at least one cholesterol synthesis inhibitor or cholesterol absorption inhibitor for at least 4 weeks.
